# EUROPEAN PATENT SPECIFICATION

(11) **EP 2 109 361 B1**
(45) Date of publication and mention of the grant of the patent: **28.12.2011**
(21) Application number: 08705168.6
(22) Date of filing: 23.01.2008
(51) Int. Cl.: A01K 67/027, C12N 15/867, A61K 48/00

(54) **A TRANSGENIC ANIMAL AND A METHOD OF PRODUCING IT**
TRANSGENES TIER UND VERFAHREN ZU SEINER HERSTELLUNG
ANIMAL TRANSGENIQUE ET PROCEDE DE PRODUCTION

(30) Priority: 25.01.2007 PL 38160507
(43) Date of publication of application: 21.10.2009
(73) Proprietor: Instytut Biologii Doswiadczalnej IM.M. Nenckiego Pan, 02-093 Warszawa (PL)
(72) Inventor: KACZMAREK, Leszek, PL-05-806 Komorow (PL); KONOPKA, Witold, PL-44-194 Knurow (PL); DUNIEC, Kamila, PL-01-460 Warszawa (PL)
(74) Representative: Witek, Rafal
(86) International application number: PCT/PL2008/000009
(87) International publication number: WO 2008/091171

(56) References cited:
- KONOPKA W. ET AL., "Application of inducible gene expression system into rat brain". ABSTRACT VIEWER, 2002, Abstract 902.2. 32nd annual meeting of the Society for Neuroscience; Orlando, FL, 2-7.11.2002 XP002478443
- AHMED B.Y. ET AL., "Efficient delivery of Cre-recombinase to adult mouse brain using adeno-associated and lentiviral vectors". ABSTRACT VIEWER, 2002, Abstract 902.6. 32nd annual meeting of the Society for Neuroscience; Orlando, FL, 2-7.11.2002 XP002478444
- UCHIDA S. ET AL.: "Tight regulation of transgene expression by tetracycline-dependent activator and repressor in brain." GENES, BRAIN AND BEHAVIOUR, vol. 5, 2006, pages 96-106, XP002478442
- MANSUY ISABELLE M ET AL: "Inducible and reversible gene expression with the rtTA system for the study of memory" NEURON, CAMBRIDGE, MA, US, vol. 21, no. 2, August 1998 (1998-08), pages 257-265, XP002205014

## Description

A transgenic animal and a method of producing it The subject of the present invention is a transgenic mammal, non human, and a method of obtaining it, which may serve to produce a favourable research tool.

For many years, animals, particularly laboratory rodents such as rats and mice, comprise models used to study the mechanisms of many physiological processes, as well as the pathogenesis of various diseases. The laboratory rat is a particular example, which has been the chief research model in neurobiology, physiology, pharmacology, toxicology and many other biomedical sciences for over 100 years. Very often, the preferred model is a transgenic animal. The production of transgenic mammals was first achieved in mice. The well known (traditional) method of obtaining transgenic transgenic mammals, for example rats, is an adaptation of earlier methods developed for mice and is based on isolating fertilized egg cells (single celled embryos) from female oviducts following induced superovulation and copulation (Hogan, B., Beddington, R., Costantini, F., and Lacy, E. (1994). Manipulating the Mouse Embryo: A Laboratory Manual, Second Edition. Cold Spring Harbor Laboratory Press, Cold Spring Harbor) and using *in vitro* methods to introduce foreign DNA into isolated single-celled embryos. Subsequently, the modified embryos are maintained until the two-celled stage and introduced into the oviducts of surrogate mothers, which are falsely pregnant females (detailed description of each stage in: Methods in Molecular Biology, Vol. 18: Transgenesis Techniques: Principles and Protocols, D.Murphy and A. Carter, Humana Press Inc., Totowa, NJ). The surrogate mothers are obtained using copulation with sterile (vasectomized) males. The state of the art is replete with examples of transgenic models obtained using the above described method or its developments.

Patent US5489742 claims a rat model of rheumatoid arthritis. The traditional method of obtaining transgenic rats was used. The lines of transgenic rats are LEW and F344. Superovulation is caused by a continuous drip of FSH (see also: Armstrong, D. T. and Opavsky, M. A. (1988 ). Superovulation of immature rats by continuous infusion of follicle-stimulating hormone. Biol. Reproduct. 39, 511-518). The embryos were transferred immediately prior to the microinjection (single-celled stage). Sprague-Dawley females copulated with vasectomized males were used as surrogate mothers.

Description EP0683226 describes only the transgenesis of mice. A standard procedure using falsely pregnant females as surrogate mothers was employed.

Application WO931420 describes an animal Alzheimer's model. The transgenesis of both rats and mice was described. In the case of mice, the technique is based on the modification of embryonic stem cells (ES). The transgenesis of rats occurs along an essentially traditional path. The Sprague-Dawley rat line was used for transgenesis. Superovulation was elicited by a single subdermal injection of PMSG without the use of hCG. The embryos were transferred immediately following the microinjection. Falsely pregnant Sprague-Dawley females were used as surrogate mothers. A procedure for obtaining rats was described in the text, but it seems apparent that only mice were obtained.

Patent description PL355353 reveals an improved method for obtaining transgenic rats using DNA microinjections into the pronucleus of the zygote.

The above mentioned methods for introducing an entire system of regulated expression into an animal using transgenesis is not useful for the central nervous system. The cited systems act simultaneously in several tissues, which means that they are useless in the study of the local effects of introduced genes, such as their activity in the brain. Thus, a real need still exists for obtaining transgenic animals which have the ability to induce local expression of a gene, particularly in nervous tissue.

The stated problem has unexpectedly been solved in the present invention.

The subject of the present invention is a transgenic, non-human animal containing :
(a) in all diploid cells, a first DNA sequence for the expression of a protein from a gene under the control of the tetracycline promoter (Tet-promoter); and
(b) in the dentate gyrus of the hippocampus, a second DNA which has been introduced by injection of a lentivirus that comprises it into the dentate gyrus of the hippocampus, said second DNA being for the expression of the rtTA gene under the control of a promoter specific for the dentate gyrus of the hippocampus, so that the expression of the rtTA induces the expression of the protein encoded by the first DNA sequence in this target tissue.

The next subject of the present invention is a method of obtaining a transgenic non-human animal, characterized in that two DNA sequences are introduced into the animal's cells, wherein:
(a) the first DNA is introduced into all diploid cells of the animal, said first DNA being for the expression of a protein from a gene under the control of the tetracycline promoter (Tet-promoter);
(b) the second DNA is introduced by injection of a lentivirus that comprises it into the dentate gyrus of the hippocampus of the animal obtained in (a), or of a progeny thereof that carries said first DNA sequence; said second DNA being for the expression of the rtTA gene under the control of a promoter specific for the dentate gyrus of the hippocampus.

Preferably, the step of introducing the first DNA involves DNA microinjection into a zygotic pronucleus.

Preferably, said promoter specific for the dentate gyrus is the EF1alpha promoter.

Preferably, the protein encoded by the first DNA is EGFP.

The description of the present invention has been supplemented with the following figures:

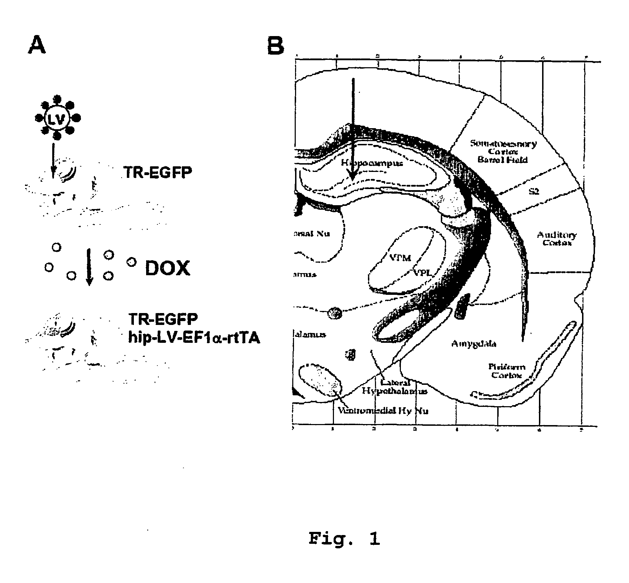

Figure 1 represents a schematic of an experiment leading to the inducible expression if EGFP in the dentate gyrus of the rat hippocampus, TetP-EGFP. (A) TetP-EGFP/hip-LV-EF1a-rtTA - TetP-EGFP transgenic rats with the lentiviral vector LV-EF1a-rtTA introduced into the dentate gyrus. DOX - doxycyclin (2 mg/ml) administered in drinking water for 14 days. (B) transverse section of a rat brain at Bregm -3,14 (Paxinos and Watson, 1998). The red arrow denotes the injection site of the lentiviral vector;

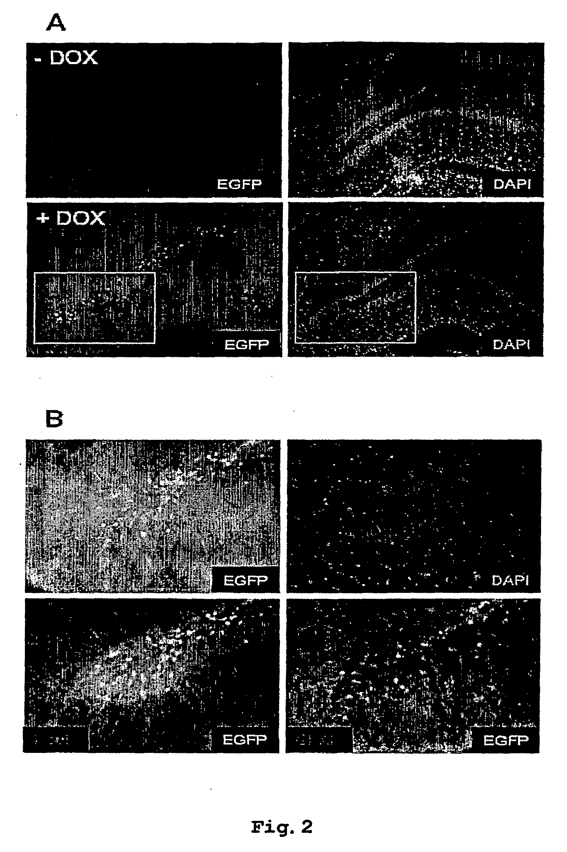

Figure 2 represents the inducible expression of the EGFP gene in the dentate gyrus of a TetP-EGFP/hip-LV-EF1α-rtTA rat. (A) Presents images of transverse sections of the brain of a TetP-EGFP rat following the introduction of the lentiviral vector LV-EF1α-rtTA. The rats were given doxycyclin (2mg/ml, 14 days) (bottom panel). The upper panel shows control tissue sections (no doxycyclin). (B) A magnification of the dentate gyrus of TetP-EGFP/hip-LV-EF1α-rtTA rats (with doxycyclin) marked in (A) (upper panel). Fluorescent staining for the NeuN neuronal marker and for astroglial cells, GFAP (bottom panel). The sections were stained with DAPI to visualise cell nuclei.

### Example 1 - Obtaining transgenic rats

In order to obtain transgenic rats, we used an improved method of microinjecting DNA into the zygote pronucleus described in patent application PL355353 submitted to the Polish Patent Office (Kaczmarek et al., 2002). The Wistar and Brown Norway (BN) rat strains were used.

In order to obtain a large number of zygotes for DNA microinjection, 5-6 week-old Wistar females were given 20 IU of PMSG (pregnant mare serum gonadotrophin) in order to stimulate ovarial follicles. After 52 h, 35 IU of hCG were given (human chorion gonadotrophibn) in order to stimulate the maturation and ovulation of oocytes. Next, the females were coupled with mature Wistar males. On the next day, fertilized oocytes were isolated from the oviducts of females which had sperm in their vaginal smears. The isolated cells were placed in a hyaluronidase solution (150 u/ml in PBS) in order to release the follicular cells surrounding the oocyte, which was then transferred into a drop of M2 medium in a plastic dish under liquid paraffin. The zygotes were incubated at 37,5 °C in an atmosphere of 5% CO2. Appropriate DNA constructs were injected into one of the zygotic pronuclei (usually the larger, male one) in a drop of M2 using a glass micropipette an electromechanical micromanipulator under a microscope. The injected single-celled embryos were maintained under optimal conditions until the following day (Hogan et al., 1986; Murphy and Carter, 1993; Pinkert, 2002).

### Example 2 - Transferring modified embryos

Zygotes obtained in Example 2 which had developed to the two-celled stage were transferred on the next day into the oviducts of 8-12 week-old surrogate mother Wistar rat females on the first day of pregnancy. The females were anaesthetized by an intraperitoneal injection of xylazine and ketamine. A portion of the rats' back was shaved and then the skin and peritoneum were incised in the rear-lateral portion of the body. The embryo transfer into the oviduct infandibulum using a glass pipette. Each female was implanted with around 15 embryos. The transgenesis made use of pregnant females, which were obtained through copulation a day earlier with BN (brown) rats. The entire progeny of this cross is brown, whereas the injected embryos (Wistar X wistar) are white (Kaczmarek et al., 2002). Among the progeny born, only the white individuals are potentially transgenic.

### Example 3 - Preparation of lentiviral vectors

A non-replicating lentiviral vector, LV-EF1α-rtTA, was produced as a result of transfecting a human kidney cell line (HEK293T) with three distinct expression plasmids: (1) a transfer plasmid (pLV-EFlα-rtTA), (2) a packing plasmid (pΔ8.7) and (3) a plasmid coding the viral capsid glycoprotein (pVSV-G). 48 hours post-transfection of 293T cells, viral particles were produced and released into the medium, which were collected along with the medium. Next, the medium was centrifuged at 1500 rpm, in order to jettison cellular contaminants. The medium with the vectors was collected and incubated with DNAse at 37 °C for 15 min, whereafter the resulting preparations were filtered through 0,45 µm Milipore filters and concentrated through ultracentrifugation (1.5 hours; 22 000 rpm; 4 °C). Next, the precipitate containing the lentiviral vectors was suspended in PBS and frozen in 10 µl aliquots at -80 °C. The titre of the LV-EF1α-rtTA lentiviral vector was estimated using Real Time-PCR and primers recognizing the short DNA sequence in the vector genome.

### Example 4 - In vivo injection of the vector

The stereotactic injections of the lentiviral vectors (LV-EF1α-rtTA) were performed on male TR-GFP transgenic rats. The rats were anaesthetized with a mixture of xylazine and ketamine. The viral vector: LV-EF1α-rtTA (3 µl; 1.5x107 particles of lentiviral vector) were injected into the dentate gyrus (Paxinos and Watson, 1998) using a Hamilton microinjector at a rate of 0.2 µl/min.

### Example 5 - Local expression in the transgenic rat from Example 4

The transgenic rats obtained in Examples 1-4 possessed the EGFP gene in their genome, under the control of a tetracycline promoter. The inclusion of the tetracycline promoter was necessary to induce the TetP promoter using doxycyclin. The lentivirus vector was used in order to introduce it into a selected brain structure. The lentiviral vector was used to introduce the rtTA gene into a selected structure, the dentate gyrus of the hippocampus of TetP-EGFP rats (Fig. 1). Such modified rats were given doxicyclin (2 mg/ml) in their drinking water for 14 days (Fig. 1). Next, the rats were killed and fixed in 4% paraformaldehyde. The presence of the green EGFP protein was ascertained using a fluorescent microscope. the expression of EGFP was observed only in the dentate gyrus of the hippocampus, at the site of injection of the lentiviral vector in rats which were given doxycyclin (Fig. 2a). The cells exhibiting EGFP presence possessed protrusions characteristic of neurons, and were collocated with the bodies of granulous cells of the dentate gyrus. Their neuronal characteristics were confirmed using a marker for neurones, NeuN. We observed no co localization of EGFP fluorescence with the GFAP astroglial marker (Fig. 2b). In control rats (without doxycyclin) we observed no EGFP expression.

## Claims

1. A transgenic, non-human animal containing .
(a) in all diploid cells, a first DNA sequence for the expression of a protein from a gene under the control of the tetracycline promoter (Tet-promoter); and
(b) in the dentate gyrus of the hippocampus, a second DNA which has been introduced by injection of a lentivirus that comprises it into the dentate gyrus of the hippocampus, said second DNA being for the expression of the rtTA gene under the control of a promoter specific for the dentate gyrus of the hippocampus, so that the expression of the rtTA induces the expression of the protein encoded by the first DNA sequence in this target tissue.

2. A method of obtaining a transgenic non-human animal, **characterized in that** two DNA sequences are introduced into the animal's cells, wherein:
(a) the first DNA is introduced into all diploid cells of the animal, said first DNA being for the expression of a protein from a gene under the control of the tetracycline promoter (Tet-promoter);
(b) the second DNA is introduced by injection of a lentivirus that comprises it into the dentate gyrus of the hippocampus of the animal obtained in (a), or of a progeny thereof that carries said first DNA sequence; said second DNA being for the expression of the rtTA gene under the control of a promoter specific for the dentate gyrus of the hippocampus.

3. The animal of claim 1 or the method of claim 2, wherein the step of introducing the first DNA involves DNA microinjection into a zygotic pronucleus.

4. The animal of claim 1 or the method of claim 2, wherein said promoter specific for the dentate gyrus is the EF1alpha promoter.

5. The animal of claim 1 or the method of claim 2, wherein the protein encoded by the first DNA is EGFP.

## Patentansprüche

1. Ein transgenes, nicht-humanes Tier, enthaltend:
(a) in allen diploiden Zellen, eine erste DNA-Sequenz für die Expression eines Proteins von einem Gen unter der Kontrolle eines Tetrazyklin-Promotors (Tet-Promotor); und
(b) im Gyrus dentatus des Hippocampus eine zweite DNA, die durch Injektion eines Lentivirus eingeführt wurde, welches es in den Gyrus dentatus trägt, wobei jene zweite DNA für die Expression des rtTA-Gens unter der Kontrolle eines Promotors steht, der für den Gyrus dentatus des Hippocampus spezifisch ist, so dass die Expression des rtTA die Expression des Proteins, das von der ersten DNA-Sequenz kodiert wird, in diesem Zielgewebe induziert.

2. Ein Verfahren zur Herstellung eines transgenen nicht-humanen Tiers, dadurch charakterisiert, dass zwei DNA-Sequenzen in die Zellen des Tiers eingeführt werden, wobei:
(a) die erste DNA in alle diploiden Zellen des Tiers eingeführt wird, wobei die erste DNA für die Expression eines Proteins unter der Kontrolle eines Tetrazyklin-Promotors (Tet-Promotor) steht;
(b) die zweite DNA durch Injektion eines Lentivirus eingeführt wird, welches es in den Gyrus dentatus des Hippocampus des in (a) hergestellten Tiers oder eines seiner Nachkommen trägt, das jene erste DNA-Sequenz trägt; wobei jene zweite DNA für die Expression des rtTA-Gens unter der Kontrolle eines Promotors steht, der für den Gyrus dentatus des Hippocampus spezifisch ist.

3. Das Tier nach Anspruch 1 oder das Verfahren nach Anspruch 2, wobei der Schritt der Einführung der ersten DNA DNA-Mikroinjektion in einen zygotischen Pronucleus umfasst.

4. Das Tier nach Anspruch 1 oder das Verfahren nach Anspruch 2, wobei jener für den Gyrus dentatus spezifische Promotor der EF1alpha-Promotor ist.

5. Das Tier nach Anspruch 1 oder das Verfahren nach Anspruch 2, wobei das von der ersten DNA kodierte Protein EGFP ist.

## Revendications

1. Animal non humain transgénique contenant :
(a) dans toutes les cellules diploïdes, une première séquence d'ADN pour l'expression d'une protéine issue d'un gène sous le contrôle du promoteur de la tétracycline (promoteur Tet) ; et
(b) dans le gyrus denté de l'hippocampe, un deuxième ADN qui a été introduit par injection d'un lentivirus le contenant dans le gyrus denté de l'hippocampe, ledit deuxième ADN étant destiné à l'expression du gène rtTa sous le contrôle d'un promoteur spécifique au gyrus denté de l'hippocampe, de sorte que l'expression du rtTa induit l'expression d'une protéine codée par la première séquence d'ADN dans ce tissu cible.

2. Procédé d'obtention d'un animal non humain transgénique, **caractérisé en ce que** deux séquences d'ADN sont introduites dans les cellules de l'animal, dans lequel :
(a) le premier ADN est introduit dans toutes les cellules diploïdes de l'animal, ledit premier ADN étant destiné à l'expression d'une protéine issue d'un gène sous le contrôle du promoteur de la tétracycline (promoteur Tet) ;
(b) le deuxième ADN est introduit par injection d'un lentivirus le contenant dans le gyrus denté de l'hippocampe de l'animal obtenu en (a), ou d'une progéniture de celui-ci qui porte ladite première séquence d'ADN ; ledit deuxième ADN étant destiné à l'expression du gène rtTa sous le contrôle d'un promoteur spécifique au gyrus denté de l'hippocampe.

3. Animal selon la revendication 1 ou procédé selon la revendication 2, dans lequel l'étape d'introduction du premier ADN implique une micro-injection d'ADN dans un pronucléus zygotique.

4. Animal selon la revendication 1 ou procédé selon la revendication 2, dans lequel ledit promoteur spécifique au gyrus denté est le promoteur EF1-alpha.

5. Animal selon la revendication 1 ou procédé selon la revendication 2, dans lequel la protéine codée par le premier ADN est EGFP.
